(19) Europäisches Patentamt
European Patent Office
Office européen des brevets

(11) EP 2 147 002 B1

(12) **EUROPÄISCHE PATENTSCHRIFT**

(45) Veröffentlichungstag und Bekanntmachung des Hinweises auf die Patenterteilung:
**22.09.2010 Patentblatt 2010/38**

(21) Anmeldenummer: **08759580.7**

(22) Anmeldetag: **14.05.2008**

(51) Int Cl.:
***C07F 3/06*** *(2006.01)* ***C07D 211/00*** *(2006.01)*

(86) Internationale Anmeldenummer:
**PCT/EP2008/055895**

(87) Internationale Veröffentlichungsnummer:
**WO 2008/138946 (20.11.2008 Gazette 2008/47)**

(54) **BISAMID-ZINKBASEN**

BISAMIDE-ZINC BASES

BASES DE BISAMIDE-ZINC

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **14.05.2007 DE 102007022490**

(43) Veröffentlichungstag der Anmeldung:
**27.01.2010 Patentblatt 2010/04**

(73) Patentinhaber: **Ludwig-Maximilians-Universität München
80359 München (DE)**

(72) Erfinder:
- **KNOCHEL, Paul
81479 München (DE)**
- **WUNDERLICH, Stefan
81377 München (DE)**

(74) Vertreter: **Sandmann, Wolfgang
Reinhard, Skuhra, Weise & Partner GbR
Patent- und Rechtsanwälte
Friedrichstrasse 31
80801 München (DE)**

(56) Entgegenhaltungen:
- **KNOCHEL, P. ; WUNDERLICH S. H.: "(tmp) 2Zns2 MgCl2s2 LiCl: eine chemoselektive Base fur die gezielte Zinkierung von empfindlichen Arenen und Heteroarenen" ANGEWANDTE CHEMIE, Bd. 119, Nr. 40, 27. August 2007 (2007-08-27), Seiten 7829-7832, XP002490914**
- **KRASOVSKIY A ET AL: "Mixed Mg/Li amides of the type R2NMgCl.cntdot.LiCl as highly efficient bases for the regioselective generation of functionalized aryl and heteroaryl magnesium compounds" ANGEWANDTE CHEMIE. INTERNATIONAL EDITION, WILEY VCH VERLAG, WEINHEIM, Bd. 45, Nr. 18, 1. Januar 2006 (2006-01-01), Seiten 2958-2961, XP002432039 ISSN: 1433-7851 in der Anmeldung erwähnt**
- **Y. KONDO, M. SHILAI, M. UCHIYAMA, T. SAKAMOTO: "TMP-Zincate as Highly Chemoselective Base for Directed Ortho Metalation" J. AM. CHEM. SOC., Nr. 121, 25. März 1999 (1999-03-25), Seiten 3539-3540, XP002490915 in der Anmeldung erwähnt**
- **HLAVINKA ET AL: "One-step deprotonation route to zinc amide and ester enolates for use in aldol reactions and Negishi couplings" TETRAHEDRON LETTERS, ELSEVIER, AMSTERDAM, Bd. 47, Nr. 29, 17. Juli 2006 (2006-07-17), Seiten 5049-5053, XP005506076 ISSN: 0040-4039 in der Anmeldung erwähnt**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 147 002 B1

## Beschreibung

Die vorliegende Erfindung betrifft Bisamid-Zinkbasen, deren Herstellung sowie die Verwendung der Zinkbasen.

Insbesondere betrifft die vorliegende Erfindung Bisamid-Zinkbasen, die zur Herstellung von Aryl- und Heteroaryl-Metallverbindungen verwendet werden können. Die Darstellung von Aryl- und Heteroaryl-Metallverbindungen kann entweder durch eine Halogen-Metallaustauschreaktion (z.B. Brom-Lithiumaustausch[1]), Insertion von elementarem Metall in eine Kohlenstoff-Halogenbindung (z.B. Zinkinsertion in eine Kohlenstoff-Iodbindung[2]) oder durch Metallierung (Deprotonierung mit Hilfe starker Basen[3]) erfolgen. Die Reaktivität der Halogene für Austauschreaktionen und Insertionen nimmt in der Reihe Iod, Brom, Chlor ab.

Aryl- oder Heteroaryl-Iodverbindungen sind jedoch im Allgemeinen teuer und meist auch nicht lange haltbar. Ein weiterer Nachteil von Austauschreaktionen und Insertionen, besonders in größerem Reaktionsmaßstab, ist die Bildung von Metallsalzen, die zum Teil gesondert entsorgt werden müssen. Daher ist es wünschenswert, eine alternative Methode zur Funktionalisierung von Aromaten und Heteroaromaten zur Verfügung zu haben, die auf einer C-H Aktivierung beruht.

Die Metallierung von Arenen ist eine der nützlichsten Umwandlungen in der organischen Synthese, da sie die regioselektive Funktionalisierung verschiedenster Arene und Heteroarene ermöglichte.[4] Dabei stellen lithiumorganische Verbindungen die reaktivsten Reagenzien dar.[5] Allerdings muss hierbei im Allgemeinen bei sehr tiefen Temperaturen gearbeitet werden, um Nebenreaktionen zu unterdrücken. Außerdem müssen teilweise die lithiumorganischen Verbindungen, wie LiTMP (TMP = 2,2,6,6-Tetramethylpiperidin) auf Grund der geringen Stabilität *in situ* dargestellt werden.[6]

Magnesiumorganische Verbindungen wie TMPMgCl·LiCl weisen eine höhere Stabilität auf, aber auch hier ist die Toleranz gegenüber sensiblen funktionellen Gruppen, wie z.B. Aldehyden und Nitrogruppen, eingeschränkt.[7]

Ein weiteres Reagenz zur Metallierung von Aromaten stellt $TMPZn\mathit{t}Bu_2Li$ dar, dessen hohe Aktivität auf einer Zinkatspezies beruht.[8] Jedoch können auch hier z.B. keine Aldehydfunktionen toleriert werden. Eine weitere Zinkbase stellt das neutrale $Zn(TMP)_2$ dar. Jedoch ist nur eine Enolatbildung von Amiden in der Literatur bekannt.[9]

Es ist daher eine Aufgabe der vorliegenden Erfindung, eine Verbindung bereitzustellen, die die Deprotonierung und Metallisierung organischer Aryl- und Heteroarylverbindungen erlaubt und die gleichzeitig eine Vielzahl funktioneller Gruppen toleriert oder durch diese nicht beeinflusst wird. Es ist eine weitere Aufgabe der vorliegenden Erfindung, ein Verfahren zum Herstellen einer solchen Verbindung bereitzustellen. Schließlich ist es eine Aufgabe der Erfindung, eine Verwendung der erfindungsgemäßen Verbindungen bereitzustellen.

Diese Aufgaben werden durch die Verbindungen und Verfahren, wie sie durch die Merkmale der unabhängigen Ansprüche definiert werden, gelöst.

Gemäß eines ersten Aspekts der vorliegenden Erfindung wird eine Verbindung der allgemeinen Formel (I)

$$(R^1R^2N)_2\text{-}Zn \cdot aMgX^1{}_2 \cdot bLiX^2 \qquad \text{(I)}$$

bereitgestellt, wobei

| | |
|---|---|
| $R^1$ und $R^2$ | unabhängig voneinander ausgewählt sind aus substituiertem oder unsubstituiertem, geradkettigem oder verzweigtem Alkyl, Alkenyl, Alkinyl oder deren Silylderivaten, und substituiertem oder unsubstituiertem Aryl oder Heteroaryl,<br>und wobei $R^1$ und $R^2$ zusammen eine Ringstruktur ausbilden können, oder $R^1$ und/oder $R^2$ Teil einer Polymerstruktur sein können; |
| $X^1{}_2$ | ein divalentes Anion ist oder zwei voneinander unabhängige monovalente Anionen sind; |
| $X^2$ | ein monovalentes Anion ist; |
| a | > 0 ist; und |
| b | > 0 ist. |

Gemäß einer bevorzugten Ausführungsform der vorliegenden Erfindung sind $R^1$ und/oder $R^2$ unabhängig voneinander ausgewählt aus geradkettigem oder verzweigtem, substituiertem oder unsubstituiertem $C_{1-20}$-Alkyl, bevorzugt $C_{1-10}$-Alkyl, noch bevorzugter $C_{1-6}$-Alkyl und am bevorzugtesten $C_{2-5}$-Alkyl.

Gemäß einer anderen bevorzugten Ausführungsform sind $R^1$ und/oder $R^2$ unabhängig voneinander ausgewählt aus Methyl, Ethyl, n-Propyl, iso-Propyl, Butyl, sec-Butyl und tert-Butyl.

Gemäß noch einer anderen bevorzugten Ausführungsform der Erfindung sind $R^1$ und/oder $R^2$ unabhängig voneinander ausgewählt aus Silylresten, bevorzugt Alkyl-substituierten Silylresten, und insbesondere bevorzugt Trimethylsilyl, Triethylsilyl, Triisopropylsilyl und t-Butyldiniethylsilyl.

In einer besonders bevorzugten Ausführungsform der vorliegenden Erfindung bilden $R^1$ und $R^2$ zusammen

eine Ringstruktur aus, bevorzugt eine Alkandiyl-Ringstruktur, und insbesondere bevorzugt 1,1,5,5-Tetramethylpentan-1,5-diyl. Durch das Ausbilden der Ringstruktur ist das Stickstoff-Atom, das an das zentrale Zink koordiniert ist, Teil einer Ringstruktur. Diese Ringstruktur kann bevorzugt 2,2,6,6-Tetramethylpiperidin sein. Es sind jedoch beliebige sekundäre Amine möglich, wobei das Stickstoff-Atom Teil einer Ringstruktur ist. Es ist daneben möglich, dass mindestens ein Stickstoffatom Teil einer polymeren Struktur ist. Dadurch wird die Zinkbase an einem Polymer immobilisiert. Bei einer insbesondere bevorzugten Ausgestaltung ist lediglich eines der beiden Stickstoff-Atome Teil einer Polymerstruktur und das andere Stickstoffatom Teil eines sckundären Amins.

In noch einer anderen, bevorzugten Ausführungsform der vorliegenden Erfindung sind $X^1$ und/oder $X^2$, unabhängig voneinander, ausgewählt aus der Gruppe, bestehend aus F; Cl; Br; I; CN; SCN; NCO; $HalO_n$, wobei n = 3 oder 4 und Hal ausgewählt ist aus Cl, Br und I; $NO_3$; $BF_4$; $PF_6$; ½ $SO_4$; H; ein Carboxylat der allgemeinen Formel $R^XCO_2$; ein Thiolat der allgemeinen Formel $SR^X$; ein Alkoholat der allgemeinen Formel $OR^X$; $R^XP(O)O_2$; $SCOR^X$; $SCSR^X$, $O_jSR^X$, wobei j = 2 oder 3 ist; oder $NO_r$, wobei r = 2 oder 3 ist; und deren Derivate;

wobei $R^X$ ein substituiertes oder unsubstituiertes $C_4$-$C_{24}$ Aryl oder ein $C_3$-$C_{24}$ Heteroaryl, das ein

oder mehrere Heteroatome, wie B, O, N, S, Se, P, oder Si, enthält; ein lineares oder verzweigtes, substituiertes oder unsubsituiertes $C_1$-$C_{20}$ Alkyl; $C_2$-$C_{20}$ Alkenyl oder $C_2$-$C_{20}$ Alkinyl; oder ein substituiertes oder unsubstituiertes $C_3$-$C_{20}$ Cycloalkyl; oder deren Derivate; oder H ist. Es ist dabei auch möglich, dass $X^1{}_2$ ein divalentes Anion, wie beispielsweise $SO_4$ ist. In einer anderen Ausgestaltung kann $X^1{}_2$ aus zwei unterschiedlichen, monovalenten Anionen aufgebaut sein, d.h. $X^{1'}$ und $X^{1''}$ liegen nebeneinander in der Formel $(R^1R^2N)_2$-Zn · aMg$X^{1'}X^{1''}$ · bLi$X^2$ vor, wobei $X^{1'}$ und $X^{1''}$ unterschiedlich, monovalente Anionen sind. Dabei können $X^{1'}$ und $X^{1''}$ unabhängig voneinander die gleiche Definition wie $X^1$ besitzen.

In einer besonders bevorzugten Weiterführung der Erfindung sind $X^1$ und/oder $X^2$ unabhängig voneinander ausgewählt aus Cl, Br und I, und bevorzugt sind $X^1$ und/oder $X^2$ Cl.

In einer anderen Ausführungsform der vorliegenden Erfindung sind a und/oder b, unabhängig voneinander, im Bereich von 0,01 - 5, bevorzugt von 0,5 - 3, noch bevorzugter von 0,9 to 2,5, und am bevorzugtesten ungefähr 2.

Ein zweiter Aspekt der vorliegenden Erfindung betrifft ein Verfahren zur Herstellung einer Verbindung, wie sie oben definiert ist, mit den Schritten:

- Bereitstellen eines Zink enthaltenden Salzes;
- Zugeben einer Magnesium-Amidbase der Formel $(R^1R^2N)$- aMg$X^1$ · bLi$X^2$,
  wobei $R^1$ und $R^2$, $X^1$ und $X^2$, sowie a und b wie oben definiert sind.

Gemäß einer bevorzugten Ausführungsform wird die Reaktion in einem Lösungsmittel durchgeführt. Dabei kann die Magnesium-Amidbase in einem Lösungsmittel gelöst sein, bevorzugt in einem Lösungsmittel, ausgewählt aus der Gruppe, bestehend aus cyclischen, linearen oder verzweigten Mono- oder Polyethern, Thioethern, Aminen, Phosphinen, und deren Derivaten, die ein oder mehrere zusätzliche Heteroatome, ausgewählt aus O, N, S und P enthalten, bevorzugt Tetrahydrofuran (THF), 2-Methyltetrahydrofuran, Dibutylether, Diethylether, tert-Butylmethylether, Dimethoxyethan, Dioxanen, bevorzugt 1,4-Dioxan, Triethylamin, Ethyldiisopropylamin, Dimethylsulfid, Dibutylsulfid; cyclischen und linearen Amiden, bevorzugt N-Methyl-2-pyrrolidon (NMP), N-Ethyl-2-pyrrolidon (NEP), N-Butyl-2-pyrrolidon (NBP), N,N-Dimethylacetamid (DMAC); cyclischen, linearen oder verzweigten Alkanen und/oder Alkenen, wobei ein oder mehrere Wasserstoffatome durch Halogen ersetzt sind, bevorzugt Dichlormethan, 1,2-Dichlorethan, $CCl_4$; Harnstoffderivaten, bevorzugt N,N'-Dimethylpropylenharnstoff (DMPU), N,N,N'N'-Tetramethylharnstoff; aromatischen, heteroaromatischen oder aliphatischen Kohlenwasserstoffen, bevorzugt Benzen, Toluen, Xylen, Pyridin, Pentan, Cyclohexan, Hexan, Heptan; Hexamethylphosphortriamid (HMPA), $CS_2$; oder Kombinationen davon.

Es ist jedoch auch möglich, dass das Zink enthaltende Salz, das auch als Zink-Salz bezeichnet wird, in einem Lösungsmittel gelöst ist. Mögliche Lösungsmittel sind die gleichen wie die oben für das Magnesium-Amid genannten Lösungsmittel. Es ist jedoch bevorzugt, dass das Zink-Salz getrocknet und ohne Lösungsmittel vorgelegt wird und anschließend das Magnesium-Amid in einem Lösungsmittel zugegeben wird. Hierdurch kann die gesamte Menge an Lösungsmittel gering gehalten werden und das Zinksalz kann leicht getrocknet werden. Zudem werden Nebenreaktionen vermindert.

Sollten während der Zugabe Ausfällungen auftreten, können diese durch Zugabe weiteren Lösungsmittels wieder gelöst werden.

Gemäß einer bevorzugten Weiterbildung der Erfindung erfolgt die Zugabe des Magnesium-Amids, das wahlweise in einem Lösungsmittel gelöst sein kann, in einem Temperaturbereich von -80°C bis 100°C, bevorzugt von -25°C bis 50°C, und am bevorzugtesten von 0°C bis 30°C. Die Zugabe erfolgt nach für einen Fachmann bekannten Verfahren. Dabei kann die Zugabe derart erfolgen, dass die Reaktionslösung sich nicht zu stark erwärmt. Die Transmetallierung von dem Magnesiumamid zum Zinkamid ist eine exotherme Reaktion. Wird daher das Magnesiumamid zu schnell oder in zu hoher Konzentration zugegeben, kann eine unerwünschte Erwärmung erfolgen. Ein Fachmann auf diesem Gebiet ist jedoch ohne übertriebene Last in der Lage, durch Routineexperimente eine optimale Zugabemenge und -zeitraum

aufzufinden. Die Zugabe kann bevorzugt kontrolliert über den Zugabezeitraum erfolgen.

In einer anderen Ausführungsform der Erfindung wird das Zink-Salz vor Zugabe der Magnesium-Amidbase getrocknet, bevorzugt im Vakuum und/oder bei einer Temperatur von über 50°C, bevorzugter über 100°C und noch bevorzugter über 150°C. Besonders bevorzugt ist eine Kombination von Vakuum und erhöhter Temperatur.

In noch einer anderen Ausführungsform der Erfindung ist die Konzentration der Magnesium-Amidbase bei Zugabe zu dem Zink-Salz im Bereich von 0,01-3 M, bevorzugt im Bereich von 0,1-1,5 M, und am bevorzugtesten im Bereich von 0,5-1,2 M. Dadurch wird eine Konzentration des Zinkamids in der resultierenden Lösung von bevorzugt 0,01-2 M, bevorzugter von 0,05-1 M, und am bevorzugtesten im Bereich von 0,1-0,6 M erreicht. Bei zu geringer Konzentration kann die Verdünnung in der Reaktionsmischung so groß sein, dass z. B. erheblich längere Reaktionszeiten bis hin zu einer ausbleibenden Reaktion auftreten können. Bei zu hoher Konzentration ist es möglich, dass die Zinkamidbase ausfällt und mit zusätzlichem Lösungsmittel wieder in Lösung gebracht werden muss. Das Ausfällen der Zinkamidbase ist für die Base selbst nicht schädlich, eine kontrollierte Abmessung von Aliquoten ist jedoch aufgrund der unbekannten Menge an ausgefallenem Amid nur schwer möglich.

Alle in dieser Beschreibung verwendeten Begriffe sollen derart verstanden werden, wie sie von einem Fachmann auf diesem Gebiet verwendet werden. Im Folgenden sollen einige Begriffe näher definiert werden.

Unter Alkyl soll hier ein gesättigter Kohlenwasserstoffrest verstanden werden, der verzweigt oder unverzweigt, d.h. geradkettig, sein kann. Bevorzugt umfasst das Alkyl 1-20 Kohlenstoffatome und bildet einen $C_1$-$C_{20}$-Alkylrest aus. Weiter bevorzugt sind Reste mit 1-10 Kohlenstoffatomen, d.h. $C_1$-$C_{10}$-Alkylreste, noch bevorzugter Reste mit 1-6 Kohlenstoffatomen, d.h. $C_1$-$C_6$-Alkylreste. Beispiele möglicher Reste sind Methyl, Ethyl, n-Propyl, iso-Propyl, n-Butyl, sec-Butyl, tert-Butyl, n-Pentyl und n-Hexyl, ohne jedoch auf diese Beispiele beschränkt zu sein.

In gleicher Weise soll unter Alkenyl ein ungesättigter Kohlenwasserstoff-Rest mit mindestens einer Kohlenstoff-Kohlenstoff-Doppelbindung verstanden werden. Bevorzugte Reste schließen $C_2$-$C_{20}$-Alkenylreste ein. Weiter bevorzugt sind $C_2$-$C_{10}$-Alkenylreste und am bevorzugtesten $C_3$-$C_6$-Alkenylreste. Ein besonders bevorzugter Rest ist der Allylrest.

Entsprechend soll unter Alkinyl ein ungesättigter Kohlenwasserstoff-Rest mit mindestens einer Kohlenstoff-Kohlenstoff-Dreifachbindung verstanden werden. Bevorzugte Alkinyl-Reste schließen $C_2$-$C_{20}$-Alkinylreste ein. Weiter bevorzugt sind $C_2$-$C_{10}$-Alkinylreste und am bevorzugtesten $C_3$-$C_6$-Alkienylreste.

Unter Silyl soll in dieser Anmeldung ein durch Alkyl, Alkenyl oder Alkinyl substituiertes Silicium-Atom verstanden werden. Ein Silylrest kann folglich durch die allgemeine

Formel -Si($R^{S1}R^{S2}R^{S3}$) dargestellt werden, wobei $R^{S1}$, $R^{S2}$ und $R^{S3}$ jeweils unabhängig voneinander ausgewählt sind aus Alkyl, Alkenyl, Alkinyl, Aryl oder Heteroaryl, wobei alle Reste wie oben definiert sind. Bevorzugte Silylreste schließen Trimethylsilyl, Triethylsilyl, Triisopropylsilyl, t-Butyldimethylsilyl und Triphenylsilyl ein.

Unter Aryl soll ein aromatisches Kohlenwasserstoff-Ringsystem verstanden werden. Bei einem Heteroaryl bzw. Hetaryl ist mindestens ein Kohlenstoffatom eines Kohlenwasserstoffsystems durch ein Heteroatom, wie beispielsweise B, N, O, S oder P, ersetzt. Beispiele für Aryl-Reste schließen Phenyl und Naphthyl ein, ohne darauf beschränkt zu sein. Beispiele für Heteroaryl schließen Pyrryl, Pyridyl, Furanyl und Thiofuryl.

Alle genannten Reste, d.h. Alkyl, Alkenyl, Alkinyl, Aryl und Heteroaryl, sind als monovalente Substituenten zu verstehen. Diese Substituenten können jedoch ihrerseits wiederum substituiert sein. Ein Fachmann wird einen möglichen Substituenten gemäß seines Fachwissens auswählen und ist in der Lage, einen Substituenten zu wählen, der nicht mit anderen Substituenten wechselwirken wird, die in dem Molekül vorliegen, und der mögliche Reaktionen nicht stören oder während dieser wechselwirken wird, insbesondere nicht bei Reaktionen, die in dieser Anmeldung beschrieben sind. Mögliche Substituenten schließen folgende ein, ohne darauf beschränkt zu sein:

- Halogene, bevorzugt Fluor, Chlor, Brom und Iod;
- Aliphatische, alicyclische, aromatische und heteroaromatische Kohlenwasserstoffe, insbesondere Alkane, Alkene, Alkine, Aryle, Arylidene, Heteroaryle und Heteroarylidene;
- Aliphatische, alicyclische, aromatische oder heteroaromatische Carbonsäureester;
- Alkohole und Alkoholate, einschließlich Hydroxylgruppen;
- Phenole und Phenolate;
- Aliphatische, alicyclische, aromatische oder heteroaromatische Ether;
- Aliphatische, alicyclische, aromatische oder heteroaromatische Amide or Amidine;
- Nitrile;
- Aliphatische, alicyclische, aromatische oder heteroaromatische Amine;
- Aliphatische, alicyclische, aromatische oder heteroaromatische Sulfides einschließlich einer Thiolgruppe;

Thiole and Thiolate.

Die Substituenten können über ein Kohlenstoffatom, ein Sauerstoffatom, ein Stickstoffatom, ein Schwefelatom oder ein Phosphoratom an die Reste gebunden sein. Als Heteroatome, z.B. in Heteroaromaten, werden bevorzugt B,

N, O, S und P verwendet.

Im folgenden soll die Erfindung ausführlicher beschrieben werden, ohne jedoch auf die speziellen Beispiele der Erfindung beschränkt zu sein.

Der Gehalt der Magesiumamidbasen und der Zinkbisamidbasen wurde unter Verwendung von Benzoesäure als Protonenquelle und 4(Phenylazo)diphenylamin als Indikator bestimmt.

Ein Beispiel für eine Zinkamidbase gemäß der Erfindung ist die neutrale Base $Zn(TMP)_2 \cdot 2LiCl \cdot 2MgCl_2$ (1). Sie erlaubt die Metallierung von Arenen und Heteroarenen unter milden Bedingungen. Hierbei kann eine Vielzahl von funktionellen Gruppen, wie z.B. Ester-, Cyano-, Nitro- und Aldehydfunktion ebenso wie Chlor- oder Bromatome, toleriert werden. $Zn(TMP)_2 \cdot 2LiCl \cdot 2MgCl_2$ lässt sich leicht in einer zweistufigen Synthese nach Schema 1 darstellen.

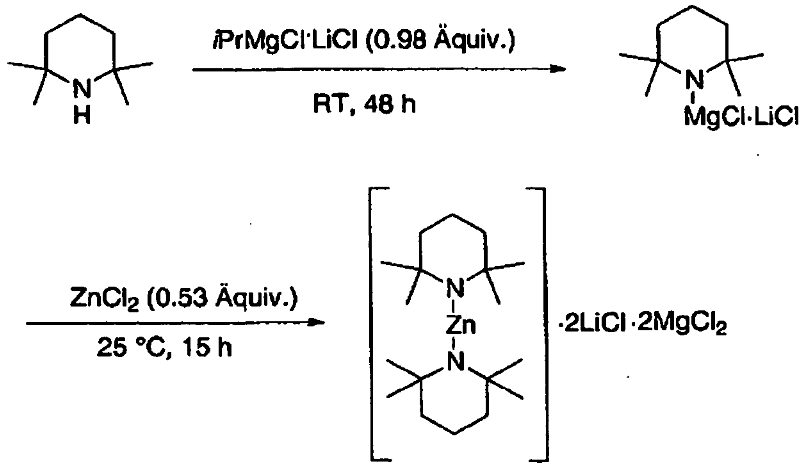


**1**: > 95%

**Schema 1**: Darstellung von $Zn(TMP)_2 \cdot 2LiCl \cdot 2MgCl_2$ (**1**).

Ausgehend von den analogen Magnesiumamiden DAMgCl·LiCl und HMDSMgCl·LiCl können auch $Zn(DA)_2 \cdot 2LiCl \cdot 2MgCl_2$ (2) und $Zn(HMDS)_2 \cdot 2LiCl \cdot 2MgCl_2$ (3) dargestellt werden (Schema 2). Dabei steht DA für Diisopropylamin und HMDS für Hexamethyldisilazan. Diese Basen weisen jedoch eine geringere Aktivität als 1 auf.

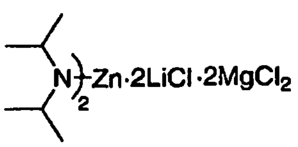

**2**

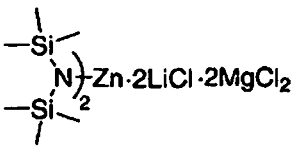

**3**

**Schema 2: Strukturen von $Zn(DA)_2 \cdot 2LiCl \cdot 2MgCl_2$ (2) und $Zn(HMDS)_2 \cdot 2LiCl \cdot 2MgCl_2$ (3).**

Bemerkenswert hierbei ist, dass nur 0.55 Äquivalente $Zn(TMP)_2 \cdot 2LiCl \cdot 2MgCl_2$ (bezogen auf das eingesetzte Startmaterial) für eine vollständige Deprotonierung benötigt werden, obwohl keine Zinkatspezies vorliegt.

Das Vorliegen eines Lithiumsalzes in der Zinkamidbase steigert die Effektivität der Zinkamidbase. Das bei der Darstellung der neutralen Zinkbase gebildete Mg-Salz führt ebenfalls zu einer Rcaktivitätssteigerung und zur verbesserten Löslichkeit der metallierten Spezies.

Die entstehenden Diaryl- und Diheteroarylzinkverbindungen können dabei in vielfältiger Weise mit Elektrophilen umgesetzt werden. Neben gängigen Abfangreaktionen mit $D_2O$ bzw. deuterierter Essigsäure oder Iod kann die Diaryl-

und Diheteroarylzinkverbindung z.B. auf Kupfer transmetalliert werden, um Allylierungen oder Acetylierungen durchzuführen (siehe Schema 3).

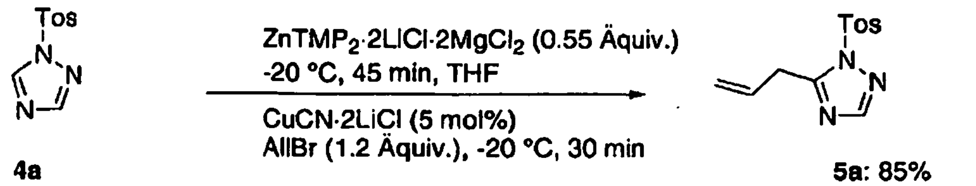


4a

5a: 85%

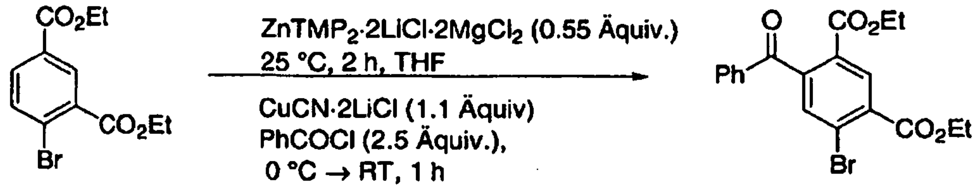


4b

5b: 83%

**Schema 3: Transmetallierung von Diaryl- und Diheteroarylzinkverbindungen auf Kupfer und anschließende Reaktionen. (RT = Raumtemperatur; Ph = Phenyl; Et = Ethyl; Tos = Tosyl)**

Des Weiteren können auch Palladium-katalysierte Kreuzkupplungen oder Reaktionen mit Heteroatomelektrophilen durchgeführt werden (Schema 4). Weitere Beispiele sind auch in Abbildung 2 angegeben.

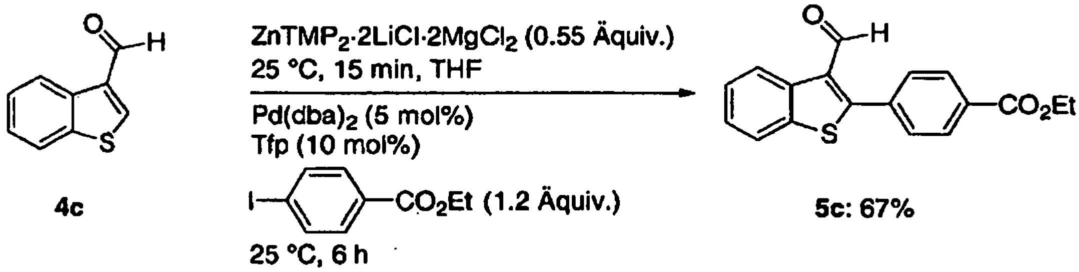


4c

5c: 67%

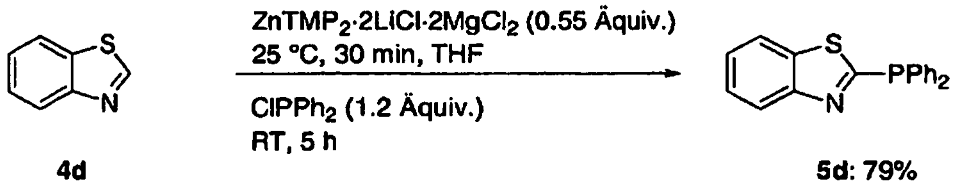


4d

5d: 79%

**Schema 4: Palladium-katalysierte Kreuzkupplung und Reaktion mit Heteroatomelektrophil.**

Das Vorliegen einer Zinkamidbase kann über verschiedene Testsysteme nachgewiesen werden. Bei dem in Schema 5 dargestellten System findet eine Reaktion mit Benzaldehyd nach Metallierung mit Zink nicht statt, wohingegen nach der Metallierung mit Magnesium eine Reaktion zu beobachten ist. Bei fehlender Umsetzung kann daher auf das Fehlen einer Mg-Spezies in der Reaktion geschlossen werden.

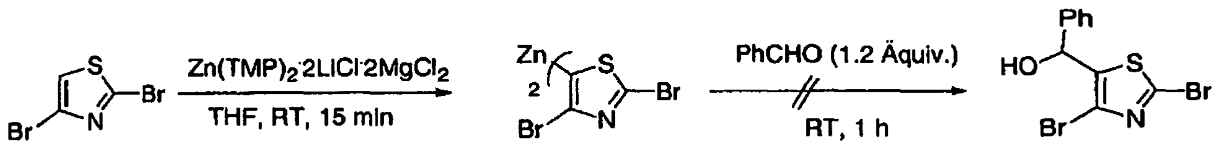


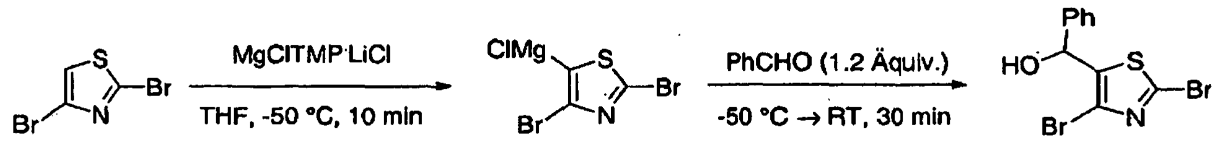


**Schema 5: Nachweis des Vorliegens einer Zinkamidbase**

Alternativ kann das Vorliegen der Zinkamidbase an einem anderen System gezeigt werden (siehe Schema 6). 2-Phenyl-[1,3,4]Oxadiazol lässt sich in kurzer Zeit deprotonieren. Jedoch zerfällt die magnesiumorganische Verbindung bei Raumtemperatur sehr schnell zu den angegebenen Produkten. Die zinkorganische Verbindung ist jedoch bei Raumtemperatur stabil und lässt sich weiter mit Elektrophilen umsetzen.

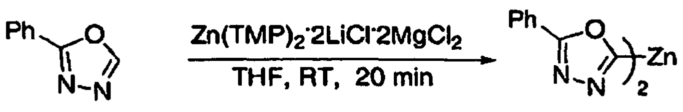


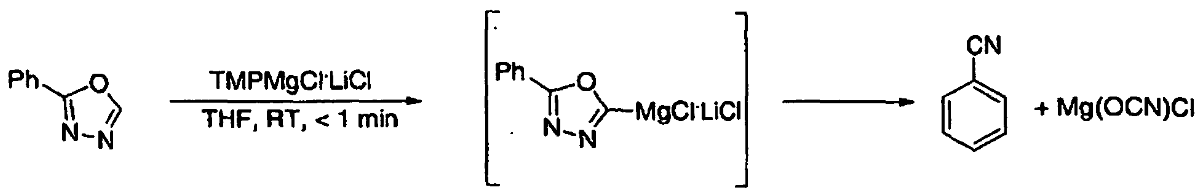


**Schema 6: Alternativer Nachweis des Vorliegens einer Zinkamidbase**

Im Folgenden wird der Einfluss diverser Salze und Amine untersucht. Dabei kann gezeigt werden, dass jede Komponente des Systems wichtig für die Reaktivität dieser Komplexbasen ist. Es konnte nachgewiesen werden, dass TMPH ein besonders aktives Amin ist. Diisopropylamin (DA) und Hexamethyldisilazan (HMDS) stellten sich als weniger reaktiv heraus.

Wie in der unten stehenden Tabelle in Schema 7 gezeigt, ist sowohl das Vorliegen von Mg als auch von Li in der Zinkamidbase wichtig. Fehlt eines dieser Metalle, so ist die Reaktivität stark vermindert oder es findet keine Reaktion statt. Es ist jedoch auch wichtig, dass eine Bisamidbase vorliegt. Liegt lediglich eine Monoamidbase, wie in Eintrag 3, vor, so kann keine Umsetzung beobachtet werden.

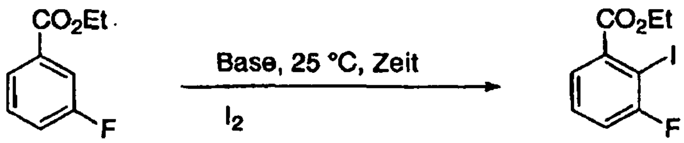


| Nr. | Base | Zeit [h] | Ausbeute |
|---|---|---|---|
| 1 | $Zn(TMP)_2 \cdot 2MgCl_2 \cdot 2LiCl$ | 12 | 80 % |
| 2 | $Zn(TMP)_2 \cdot 2MgBr_2 \cdot 2LiCl$ | 12 | 79 % |
| 3 | $Zn(TMP)Cl \cdot MgCl_2 \cdot 2LiCl$ | 12 | sehr langsame Reaktion |
| 4 | $Zn(TMP)_2 \cdot 2LiCl$ | 12 | keine Reaktion |
| 5 | $Zn(TMP)_2 \cdot$ | 12 | keine Reaktion |

**Schema 7**: Abhängigkeit der Reaktionsgeschwindigkeit von diversen Salzen.

Die unterschiedliche Reaktivität verschiedener Amide kann in einem System mit 2-Phenyl-[1,3,4]Oxadiazol gezeigt werden. Die allgemeine Reaktion und die erzielten Ausbeuten sind unten angegeben.

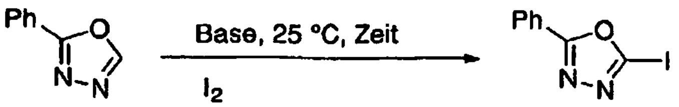


| Nr. | Base | Zeit [h] | Ausbeute |
|---|---|---|---|
| 1 | $Zn(TMP)_2 \cdot 2MgCl_2 \cdot 2LiCl$ | 0.3 | 80% |
| 2 | $Zn(DA)_2 \cdot 2MgCl_2 \cdot 2LiCl$ | 6 | 75% |
| 3 | $Zn(HMDS)_2 \cdot 2MgCl_2 \cdot 2LiCl$ | 9 | 73% |

**Schema 8**: Abhängigkeit von unterschiedlichen Amiden

Aus obigen Ergebnissen ist ersichtlich, dass alle drei Base eine Reaktivität zeigen, die TMP-Base jedoch die höchste Reaktivität zeigt.

Eine weitere Steigerung der Reaktivität der Zinkamidbasen kann durch den Einsatz von Mikrowellenstrahlung erfolgen. Insbesondere bei aromatischen Systemen mit sehr langen Reaktionszeiten auf Grund der niedrigen CH-Acidität der Protonen, wie beispielhaft in Schema 9 dargestellt, kann durch den Einsatz von Mikrowellenstrahlung die Reaktionszeit deutlich verkürzt werden.

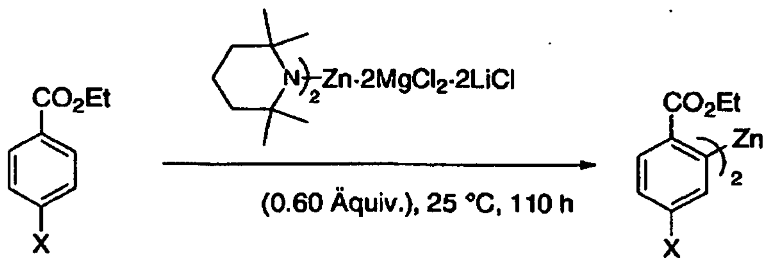


X = Br, Cl

X = Br, Cl

**Schema 9: Aromaten mit sehr langen Metallierungszeiten.**

Eine Ausführungsform der Verwendung der Verbindungen der Formel I ist eine Umsetzung mit einem Elektrophil, wobei zunächst ein CH-acides Substrat mit einer Verbindung der Formel I deprotniert und anschließend das deprotonierte Substrat mit einem Elektrophil umgesetzt wird. In einer vorteilhaften Weiterführung dieser Verwendung wird die Deprotonierung des CH-aciden Substrates unter Mikrowellenbestrahlung durchgeführt. Besonders vorteilhaft wird dabei Mikrowellenbestrahlung mit einer Energie im Bereich von 1 - 500 W, bevorzugt im Bereich von 20 - 300 W, weiter bevorzugt im Bereich von 40 - 200 W, und am bevorzugtesten im Bereich von 80-150 W verwendet.

Wird beispielsweise die Umsetzung, wie sie in Schema 9 dargestellt ist, in der Mikrowelle durchgeführt, so lässt sich die Dauer der Metallierung enorm verkürzen (siehe Schema 10). Bei den in Schema 10 gezeigten Beispielen dauert nun die Metallierung nicht mehr 110 Stunden, sondern lediglich 2 Stunden, d.h. weniger als 2% der ursprünglichen Zeit. Dabei können, nach Abfangreaktionen mit verschiedenen Elektrophilen, die Produkte in guten bis exzellenten Ausbeuten erhalten werden. Somit lässt sich durch den Einsatz von Mikrowellenstrahlung eine enorme Zeitersparnis erzielen bei gleichzeitig zumindest gleich bleibenden Ausbeuten.

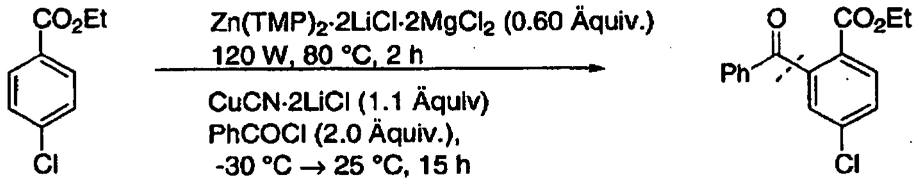


**6a** **7a**: 86%

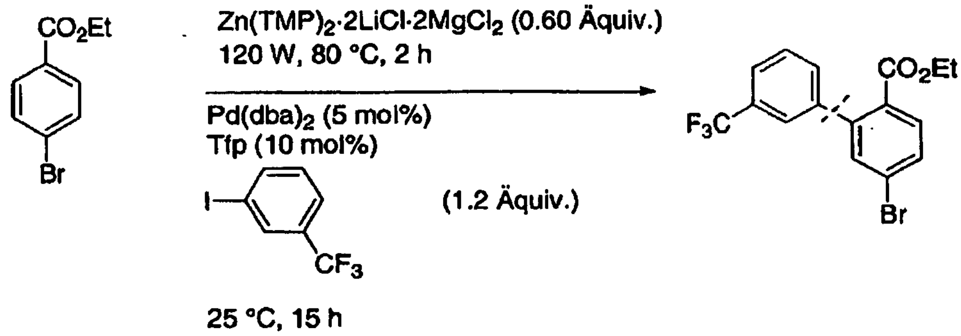


**6b** **7b**: 76%

**Schema 10**: Mikrowellen-beschleunigte Metallierungsreaktionen und anschließende Umsetzung mit Elektrophilen.

Auch an Substraten mit Cyanogruppen lässt sich leicht eine Mikrowellen-beschleunigte Metallierung durchführen. Die Verwendung der Mikrowellenstrahlung erlaubt nun sogar die Metallierung von Ethylbenzoat und *N,N*-Diethylbenzamid mittels $Zn(TMP)_2 \cdot 2LiCl \cdot 2MgCl_2$ (siehe Schema 11). Diese Substrate zeigten unter herkömmlichen Bedingungen (Reaktion bei Raumtemperatur bzw. Erhitzen in einem Ölbad auf 55 °C) keine Reaktion.

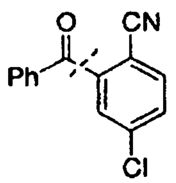

120 W, 90 °C, 3.5 h; 69%

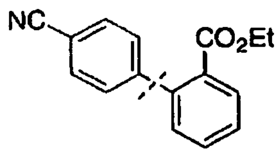

120 W, 120 °C, 5 h; 82%

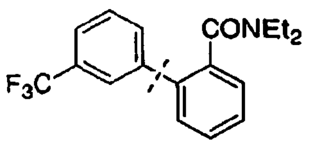

120 W, 120 °C, 5 h; 85%

**Schema 11**: Weitere Bespiele Mikrowellen-beschleunigter Metallierungsreaktionen.

Auch weniger reaktive heterozyklische Systeme wie Benzothiophen (8) lassen sich leicht mit dieser neuen Methode funktionalisieren (Schema 12).

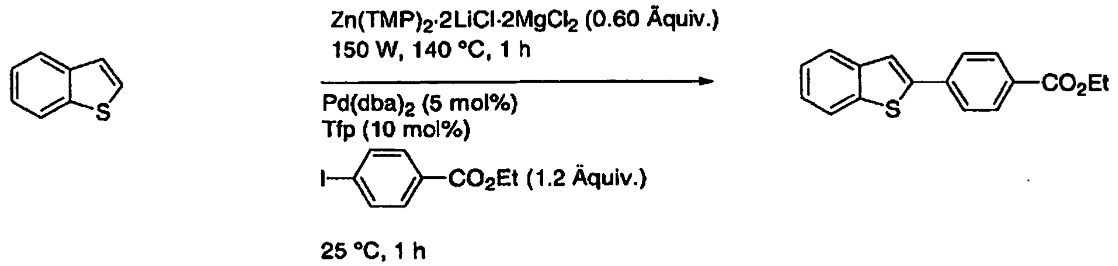


8

9: 95%

**Schema 12: Mikrowellen-beschleunigte Metallierungsreaktion an Benzothiophen und anschließende Umsetzung mit Elektrophil.**

Somit lässt sich feststelen, dass die Mikrowellen-beschleunigte Metallierung ein äußerst nützliches Werkzeug zur Funktionalisierung, insbesondere von Aromaten und Heteroaromaten, darstellt, da selbst reaktionsträge Substrate effizient und schnell umgesetzt werden können. Abschließend ist zu sagen, dass mit dieser vorgestellten Methode ein großer Schritt gelungen ist, hohe Reaktivität mit Selektivität und Toleranz gegenüber sensiblen funktionellen Gruppen zu verbinden.

Beispiele

Beispiel 1: Typische Arbeitvorschrift zur Darstellung von $Zn(TMP)_2·2LiCl·2MgCl_2$ (1):

$ZnCl_2$ (21.0 mmol, 2.86 g) wird in einem 100 mL Schlenk-Kolben unter Rühren im Ölpumpenvakuum bei 150°C für 5 h getrocknet. Nach dem Abkühlen wird bei Raumtemperatur langsam TMPMgCl·LiCl (40.0 mmol, 1.11 M, 36.0 mL) in Tetrahydrofuran (THF) gelöst zugegeben und 15 h bei Raumtemperatur gerührt. Eventuell auftretende Niederschläge von $Zn(TMP)_2·2LiCl·2MgCl_2$ können dabei durch Zugabe von trockenem THF wieder gelöst werden.

Beispiel 2: Typische Arbeitsvorschrift für die Darstellung von **5c**:

In einen trockenen und mit Argon befüllten 25-mL-Schlenk-Kolben mit magnetischem Rührkern und Septum wurde 1-Benzothiophen-3-carbaldehyd (4c) (164 mg, 1.00 mmol) in 1.0 mL trockenem THF vorgelegt und tropfenweise mit $Zn(TMP)_2·2LiCl·2MgCl_2$ in THF gelöst (1.5 mL, 0.37 M, 0.55 Äquivalente) bei Raumtemperatur versetzt. Nach 40 min war die Metallierung vollständig (GC-Analyse von Reaktionsproben, die mit einer Lösung von $I_2$ in THF versetzt wurden, zeigte einen Umsatz von mehr als 98% an.). Dann wurde langsam eine Lösung von 4-$EtO_2CPh$-I (331 mg, 1.2 Äquivalente), $Pd(dba)_2$ (5 mol%) (dba=Dibenzylidenaceton) und Triortho-furylphosphin (Tfp) (10 mol%) in trockenem THF (2.5 mL) zugegeben. Nach 5 h wurde die Reaktion durch Zugabe von gesättigter $NH_4Cl$-Lösung (10 mL) beendet. Die wässrige Phase wurde mit Ethylacetat (5 x 10 mL) extrahiert, über $MgSO_4$ getrocknet und unter vermindertem Druck eingeengt. Das Rohprodukt wurde durch Filter-Säulenchromatographie gereinigt (Kieselgel; $CH_2Cl_2$ /Pentan 1:1); man erhielt 5c (208 mg, 67%) als gelben, kristallinen Feststoff.

Beispiel 3: Typische Arbeitsvorschrift für die Darstellung von 5h:

In einen trockenen und mit Argon befüllten 25-mL-Schlenk-Kolben mit magnetischem Rührern und Septum wurden 2-Nitrobenzofuran (4h) (163 mg, 1.00 mmol) in 1.0 mL trockenem THF vorgelegt, auf -30°C gekühlt und tropfenweise mit $Zn(TMP)_2·2LiCl·2MgCl_2$ in THF gelöst (1.5 mL, 0.37 M, 0.55 Äquivalente) versetzt. Nach 90 min war die Metallierung vollständig (GC-Analyse von Reaktionsproben, die mit einer Lösung von $I_2$ in THF versetzt wurden, zeigte einen Umsatz von mehr als 98% an.) und es wurde langsam $CH_3COOD$ (10 Äquiv.) in $D_2O$ zugegeben und 10 min gerührt. Die Reaktion wurde durch Zugabe von gesättigter $NH_4Cl$-Lösung (10 mL) beendet. Die wässrige Phase wurde mit Ethylacetat (5 x 10 mL) extrahiert, über $MgSO_4$ getrocknet und unter vermindertem Druck eingeengt. Das Rohprodukt wurde durch Filter-Säulenchromatographie gereinigt (Kieselgel; $CH_2Cl_2$); man erhielt **5h** (133 mg, 81%) als gelben, kristallinen Feststoff.

Beispiel 4: Typische Arbeitvorschrift zur Darstellung von 7b:

In einen trockenen und mit Argon befüllten 10-mL-Mikrowellen-Rohr mit magnetischem Rührkern und Septum wurde 6b (458 mg, 2.00 mmol) in 1.0 mL trockenem THF vorgelegt und mit $Zn(TMP)_2 \cdot 2LiCl \cdot 2MgCl_2$ in THF versetzt (3.2 mL, 0.37 M, 0.60 Äquivalente) bei 25 °C versetzt. Das Mikrowellen-Rohr wurde verschlossen und die Reaktion gestartet. Nach 2 h in der Mikrowelle (120 W, 80 °C) war die Metallierung vollständig (GC-Analyse von Reaktionsproben, die mit einer Lösung von $I_2$ in THF versetzt wurden, zeigte einen Umsatz von mehr als 98% an.). Nach dem Abkühlen der Reaktionsmischung auf Raumtemperatur wurde diese in eine Lösung von 3-$CF_3$-$C_6H_4$-I (680 mg, 1.2 Äquivalente), Pd $(dba)_2$ (5 mol%) und Tfp (10 mol%) in trockenem THF (2.5 mL) gegeben. Nach 15h Rühren bei 25 °C wurde die Reaktion durch Zugabe von gesättigter $NH_4Cl$-Lösung (30 mL) beendet. Die wässrige Phase wurde mit Ethylacetat (5 x 30 mL) extrahiert, über $MgSO_4$ getrocknet und unter vermindertem Druck eingeengt. Das Rohprodukt wurde durch Filter-Säulenchromatographie gereinigt (Kieselgel; $Et_2O$ /Pentan 1:15); man erhielt 7b (680 mg, 76%) als gelbes Öl.

Gemäß den typischen Arbeitsvorschriften der Beispiele 2 und 3 wurden folgende Produkte synthetisiert.

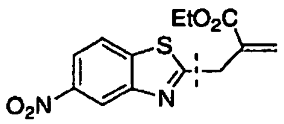

**5e**: 76[a]

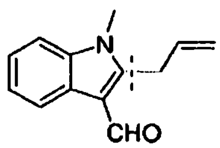

**5f**: 71%[a]

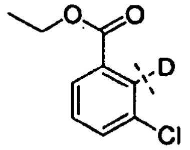

**5g**: 84%[b]

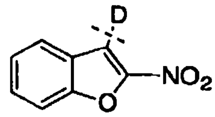

**5h**: 82%[b]

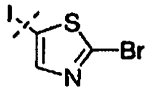

**5i**: 84%[c]

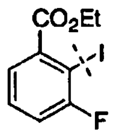

**5j**: 70%[c]

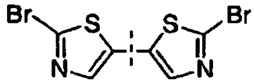

**5k**: 91%[d]

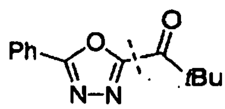

**5l**: 71%[e]

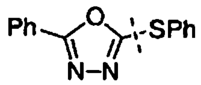

**5m**: 75%[f]

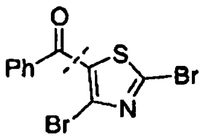

**5n**: 84%[e]

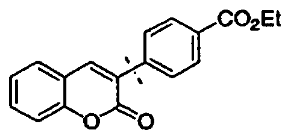

**5o**: 85%[g]

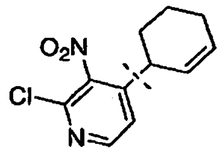

**5p**: 82%[a]

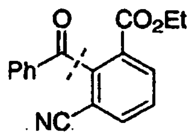

**5q**: 73%[e]

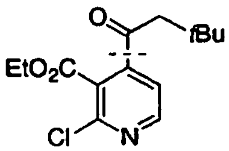

**5r**: 75%[e]

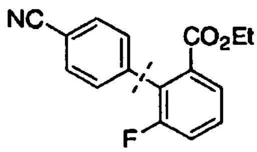

**5s**: 64%[g]

**Abbildung 2: Anmerkungen: a) Verwendung von CuCN·2LiCl (5 mol%); b) $CH_3COOD$ (10 Äquiv.) in $D_2O$ wurde als Abfangreagenz verwendet; c) $I_2$ (1.0 Äquiv) wurde als Elektrophil verwendet; d) Verwendung von Chloranil (1.2 Äquiv), um die Homokupplung durchzuführen; e) Verwendung von CuCN·2LiCl (1.1 Äquiv.); f) $PhSSO_2Ph$ wurde als Elektrophil verwendet. g) Verwendung von $Pd(dba)_2$ (5 mol-%) und Tfp (10 mol%) als Katalysator**

**Literatur**

1 a) T. Bach, S. Heuser, J. Org. Chem. 2002, 67, 5789; b) A. Dondoni, G. Fantin, M. Foagnolo, A. Medici, P. Pedrini, J. Org. Chem. 1998, 53, 1748.
2 M. Gaudemar, Bull. Soc. Chim. Fr. 1962, 974; b) P. Knochel, M. C. P. Yeh, S. C. Berk, J. Talbert, J. Org. Chem. 1988, 53, 2390.
3 V. Snieckus, Chem. Rev. 1990, 90, 879.
4 a) M. Schlosser , Angew. Chem. 2005, 117, 380; Angew. Chem. Int. Ed. 2005, 44, 376; b) C.-C. Chang, M. S. Ameerunisha, Coord. Chem. Rev. 1999, 189, 199; c) J. Clayden, Organolithiums: Selectivity for Synthesis (Hrsg.: J. E. Baldwin, R. M. Williams), Elsevier, 2002.
5 "The Preparation of Organolithium Reagents and Intermediates": F. Leroux, M. Schlosser , E. Zohar, I. Marek, Chemistry of Organolithium Compounds (Hrsg.: Z. Rappoport, I. Marek), Wiley, New York, 2004, Kap.1, S. 435.
6 I. E. Kopka, Z. A. Fataftah, M. W. Rathke, J. Org. Chem. 1987, 52, 448.
7 A. Krasovskiy, V. Krasovskaya, P. Knochel, Angew. Chem. Int. Ed. 2006, 45, 2958; Angew. Chem. 2006, 118, 3024.
8 Y. Kondo, M. Shilai, M. Uchiyama, T. Sakamoto, J. Am. Chem. Soc. 1999, 121, 3539; b) W. Clegg, S. Dale, R. W. Harrington, E. Hevia, G. W. Honeyman, R.E. Mulvey, Angew. Chem. Int. Ed. 2006, 45, 2374; Angew. Chem. 2006, 118, 2434; c) W. Clegg, S. Dale, A. Drummond, E. Hevia, G. W. Honeyman, R.E. Mulvey, J. Am. Chem. Soc. 2006, 128, 7434; d) D. A. Armstrong, W. Clegg, S. Dale, E. Hevia, L. Hogg, G. W. Honeyman, R. E. Mulvey, Angew. Chem. Int. Ed. 2006, 45, 3775; Angew. Chem. 2006, 118, 3859.
9 M. Hlavinka, J. Hagadorn, Tett. Lett., 2006, 47, 5049.

## Patentansprüche

1. Verbindung der allgemeinen Formel (I)

$$(R^1R^2N)_2\text{-Zn} \cdot aMgX^1{}_2 \cdot bLiX^2 \qquad \text{(I)}$$

wobei
$R^1$ und $R^2$ unabhängig voneinander ausgewählt sind aus substituiertem oder unsubstituiertem, geradkettigem oder verzweigtem Alkyl, Alkenyl, Alkinyl oder deren Silylderivaten, und substituiertem oder unsubstituiertem Aryl oder Heteroaryl, und wobei $R^1$ und $R^2$ zusammen eine Ringstruktur ausbilden können, oder $R^1$ und/oder $R^2$ Teil einer Polymerstruktur sein können;
$X^1{}_2$ ein divalentes Anion ist oder zwei voneinander unabhängige monovalente Anionen sind;
$X^2$ ein monovalentes Anion ist;
$a > 0$ ist; und
$b > 0$ ist.

2. Verbindung nach Anspruch 1, **dadurch gekennzeichnet, dass** $R^1$ und/oder $R^2$ unabhängig voneinander ausgewählt sind aus geradkettigem oder verzweigtem, substituiertem oder unsubstituiertem $C_{1-20}$-Alkyl, bevorzugt $C_{1-10}$-Alkyl, noch bevorzugter $C_{1-6}$-Alkyl und am bevorzugtesten $C_{2-5}$-Alkyl.

3. Verbindung nach einem oder mehreren der vorhergehenden Ansprüche, **dadurch gekennzeichnet, dass** $R^1$ und/oder $R^2$ unabhängig voneinander ausgewählt sind aus Silyltesten, bevorzugt Alkyl-substituierten Silylresten, und insbesondere bevorzugt Trimethylsilyl, Triethylsilyl, Triisopropylsilyl und t-Butyldimethylsilyl.

4. Verbindung nach Anspruch 1, **dadurch gekennzeichnet, dass** $R^1$ und $R^2$ zusammen eine Ringstruktur ausbilden, bevorzugt eine Alkandiyl-Ringstruktur, und insbesondere bevorzugt 1,1,5,5-Tetramethylpentan-1,5-diyl ist.

5. Verbindung nach einem oder mehreren der vorhergehenden Ansprüche, **dadurch gekennzeichnet, dass** $X^1$ ausgewählt ist aus der Gruppe, bestehend aus F; Cl; Br; I; CN; SCN; NCO; $HalO_n$, wobei n = 3 oder 4 und Hal ausgewählt ist aus Cl, Br und I; $NO_3$; $BF_4$; $PF_6$; ½ $SO_4$; H; ein Carboxylat der allgemeinen Formel $R^XCO_2$; ein Thiolat der allgemeinen Formel $SR^X$; ein Alkoholat der allgemeinen Formel $OR^X$; $R^XP(O)O_2$; $SCOR^X$; $SCSR^X$; $O_jSR^X$, wobei j = 2 oder 3 ist; oder $NO_r$, wobei r = 2 oder 3 ist; und deren Derivate; wobei $R^X$ ein substituiertes oder unsubstituiertes $C_4$-$C_{24}$ Aryl oder ein $C_3$-$C_{24}$ Heteroaryl, das ein oder mehrere Heteroatome, wie B, O, N, S, Se, P, oder Si, enthält; ein lineares oder verzweigtes, substituiertes oder unsubsituiertes $C_1$-$C_{20}$ Alkyl; $C_2$-$C_{20}$ Alkenyl oder $C_2$-$C_{20}$ Alkinyl;

oder ein substituiertes oder unsubstituiertes $C_3$-$C_{20}$ Cycloalkyl; oder deren Derivate; oder H ist.

**6.** Verbindung nach einem oder mehreren der vorhergehenden Ansprüche, **dadurch gekennzeichnet, dass** $X^2$ ausgewählt ist aus der Gruppe, bestehend aus F; Cl; Br; I; CN; SCN; NCO; $HalO_n$, wobei n = 3 oder 4 und Hal ausgewählt ist aus Cl, Br und I; $NO_3$; $BF_4$; $PF_6$; ½ $SO_4$; H; ein Carboxylat der allgemeinen Formel $R^XCO_2$; ein Disilazid der allgemeinen Formel $(R^X{}_3Si)_2N$; ein Thiolat der allgemeinen Formel $SR^X$; ein Alkoholat der allgemeinen Formel $OR^X$; $R^XP(O)O_2$; $SCOR^X$; $SCSR^X$; ein Amin der allgemeinen Formel $R^XNH$; ein Dialkyl- oder Diarylamin der allgemeinen Formel $R^X{}_2N$, wobei $R^X$ wie unten definiert ist oder $R^X{}_2N$ ein cyclisches Alkylamin darstellt; ein Phosphin der allgemeinen Formel $PR^X{}_2$, wobei $R^X$ wie unten definiert ist oder $PR^X{}_2$ ein cyclisches Phosphin darstellt; $O_jSR^X$, wobei j = 2 oder 3 ist; oder $NO_r$, wobei r = 2 oder 3 ist; und deren Derivate; wobei $R^X$ ein substituiertes oder unsubstituiertes $C_4$-$C_{24}$ Aryl oder ein $C_3$-$C_{24}$ Heteroaryl, das ein oder mehrere Heteroatome, wie B, O, N, S, Se, P, oder Si, enthält; ein lineares oder verzweigtes, substituiertes oder unsubsituiertes $C_1$-$C_{20}$ Alkyl; $C_2$-$C_{20}$ Alkenyl oder $C_2$-$C_{20}$ Alkinyl; oder ein substituiertes oder unsubstituiertes $C_3$-$C_{20}$ Cycloalkyl; oder deren Derivate; oder H ist.

**7.** Verbindung nach einem oder mehreren der vorhergehenden Ansprüche, **dadurch gekennzeichnet, dass** a im Bereich von 0,01 - 5, bevorzugt von 0,5 - 3, noch bevorzugter von 0,9 to 2,5, und am bevorzugtesten ungefähr 2 ist.

**8.** Verbindung nach einem oder mehreren der vorhergehenden Ansprüche, **dadurch gekennzeichnet, dass** b im Bereich von 0,01 - 5, bevorzugt von 0,5 - 3, noch bevorzugter von 0,9 to 2,5, und am bevorzugtesten ungefähr 2 ist.

**9.** Verfahren zur Herstellung einer Verbindung gemäß einem oder mehreren der vorhergehenden Ansprüche mit den Schritten:

- Bereitstellen eines Zink enthaltenden Salzes;
- Zugeben einer Magnesium-Amidbase der Formel $(R^1R^2N)$- $aMgX^1 \cdot bLiX^2$, wobei

$R^1$ und $R^2$ unabhängig voneinander ausgewählt sind aus substituiertem oder unsubstituiertem, geradkettigem oder verzweigen Alkyl, Alkenyl, Alkinyl oder deren Silylderivaten, substituiertem oder unsubstituiertem Aryl oder Heteroaryl, und wobei $R^1$ und $R^2$ zusammen eine Ringstruktur ausbilden können, oder $R^1$ und/oder $R^2$ Teil einer Polymerstruktur sein können;
$X^1{}_2$ ein divalentes Anion oder zwei voneinander unabhängige monovalente Anionen ist;
$X^2$ ein monovalentes Anion ist;
a > 0; und
b > 0 ist.

**10.** Verfahren nach Anspruch 9, **dadurch gekennzeichnet, dass** die Magnesium-Amidbase in einem Lösungsmittel gelöst ist, bevorzugt in einem Lösungsmittel, ausgewählt aus der Gruppe, bestehend aus cyclischen, linearen oder verzweigten Mono- oder Polyethern, Thioethern, Aminen, Phosphinen, und deren Derivaten, die ein oder mehrere zusätzliche Heteroatome, ausgewählt aus O, N, S und P enthalten, bevorzugt Tetrahydrofuran (THF), 2-Methyltetrahydrofuran, Dibutylether, Diethylether, tert-Butylmethylether, Dimethoxyethan, Dioxanen, bevorzugt 1,4-Dioxan, Triethylamin, Ethyldiisopropylamin, Dimethylsulfid, Dibutylsulfid; cyclischen und linearen Amiden, bevorzugt N-Methyl-2-pyrrolidon (NMP), N-Ethyl-2-pyrrolidon (NEP), N-Butyl-2-pyrrolidon (NBP), N,N-Dimethylacetamid (DMAC); cyclischen, linearen oder verzweigten Alkanen und/oder Alkenen, wobei ein oder mehrere Wasserstoffatome durch Halogen ersetzt sind, bevorzugt Dichlormethan, 1,2-Dichlorethan, $CCl_4$; Hamstoffderivaten, bevorzugt N,N'-Dimethylpropylenharnstoff (DMPU), N,N,N'N'-Tetramethylharnstoff; aromatischen, heteroaromatischen oder aliphatischen Kohlenwasserstoffen, bevorzugt Benzen, Toluen, Xylen, Pyridin, Pentan, Cyclohexan, Hexan, Heptan; Hexamethylphosphortriamid (HMPA), $CS_2$; oder Kombinationen davon.

**11.** Verfahren nach einem oder mehreren der Ansprüche 9-10, **dadurch gekennzeichnet, dass** die Zugabe in einem Temperaturbereich von -80°C bis 100°C, bevorzugt von -25°C bis 50°C, und am bevorzugtesten von 0°C bis 30°C erfolgt.

**12.** Verfahren nach einem oder mehreren der Ansprüche 9-11, **dadurch gekennzeichnet, dass** das Zink enthaltende Salz vor Zugabe der Magnesium-Amidbase getrocknet wird, bevorzugt im Vakuum und/oder bei einer Temperatur von über 50°C, bevorzugter über 100°C und noch bevorzugter über 150°C.

**13.** Verfahren nach einem oder mehreren der Ansprüche 9-12, **dadurch gekennzeichnet, dass** die Konzentration der Magnesium-Amidbase bei Zugabe zu dem Zink enthaltenden Salz im Bereich von 0,01-3 M, bevorzugt im Bereich

von 0,1-1,5 M, und am bevorzugtesten im Bereich von 0,5-1,2 M ist.

**14.** Verfahren nach einem oder mehreren der Ansprüche 9-13, **dadurch gekennzeichnet, dass** die Konzentration der resultierenden Zinkamidbase im Bereich von 0,01-2 M, bevorzugt im Bereich von 0,05-1 M, und am bevorzugtesten im Bereich von 0,1-0,6 M ist.

**15.** Verfahren nach einem oder mehreren der Ansprüche 9-14, **dadurch gekennzeichnet, dass** das Zink enthaltende Salz vor Zugabe der Magnesiumamidbase in einem Lösungsmittel gelöst wird.

**Claims**

**1.** A compound of the general formula (I)

$$(R^1R^2N)_2\text{-Zn} \cdot a\text{MgX}^1{}_2 \cdot b\text{LiX}^2 \qquad \text{(I)}$$

wherein
$R^1$ and $R^2$ are independently selected from substituted or unsubstituted, linear or branched alkyl, alkenyl, alkynyl or silyl derivatives thereof, and substituted or unsubstituted aryl or heteroaryl,
and wherein $R^1$ and $R^2$ can form together a ring structure, or $R^1$ and/or $R^2$ can be part of a polymer structure;
$X^1{}_2$ is a divalent anion or two monovalent anions that are independent from each other; $X^2$ is a monovalent anion;
a is > 0; and
b is > 0.

**2.** The compound according to claim 1, **characterised in that** $R^1$ and/or $R^2$ are each independently selected from linear or branched, substituted or unsubstituted $C_{1\text{-}20}$-alkyl, preferably $C_{1\text{-}10}$-alkyl, more preferably $C_{1\text{-}6}$-alkyl and most preferably $C_{2\text{-}5}$-alkyl.

**3.** The compound according to one or more of the preceding claims, **characterised in that** $R^1$ and/or $R^2$ are independently selected from silyl radicals, preferably alkylsubstituted silyl radicals and more preferably trimethylsilyl, triethylsilyl, triisopropylsilyl and t-butyldimethylsilyl.

**4.** The compound according to claim 1, **characterised in that** $R^1$ and $R^2$ form together a ring structure, preferably an alkanediyl ring structure and more preferably 1,1,5,5-tetramethylpentane-1,5-diyl.

**5.** The compound according to one or more of the preceding claims, **characterised in that** $X^1$ is selected from the group consisting of F; Cl; Br; I; CN; SCN; NCO; $HalO_n$, wherein n = 3 or 4 and Hal is selected from Cl, Br and I; $NO_3$; $BF_4$; $PF_6$; ½ $SO_4$; H; a carboxylate of the general formula $R^XCO_2$; a thiolate of the general formula $SR^X$; an alcoholate of the general formula $OR^X$; $R^XP(O)O_2$; $SCOR^X$; $SCSR^X$; $O_jSR^X$, wherein j = 2 or 3; or $NO_r$, wherein r = 2 or 3; and derivatives thereof;
wherein $R^X$ is a substituted or unsubstituted $C_4$-$C_{24}$ aryl or a $C_3$-$C_{24}$ heteroaryl which contains one or more heteroatoms, such as B, O, N, S, Se, P, or Si; a linear or branched, substituted or unsubstituted $C_1$-$C_{20}$ alkyl; $C_2$-$C_{20}$ alkenyl or $C_2$-$C_{20}$ alkynyl; or a substituted or unsubstituted $C_3$-$C_{20}$ cycloalkyl; or derivatives thereof; or H.

**6.** The compound according to one or more of the preceding claims, **characterised in that** $X^2$ is selected from the group consisting of F; Cl; Br; I; CN; SCN; NCO; $HalO_n$, wherein n = 3 or 4 and Hal is selected from Cl, Br and I; $NO_3$; $BF_4$; $PF_6$; ½ $SO_4$; H; a carboxylate of the general formula $R^XCO_2$; a disilazide of the general formula $(R^X{}_3Si)_2N$; a thiolate of the general formula $SR^X$; an alcoholate of the general formula $OR^X$; $R^XP(O)O_2$; $SCOR^X$; $SCSR^X$; an amine of the general formula $R^XNH$; a dialkyl- or diarylamine of the general formula $R^X{}_2N$, wherein $R^X$ is as defined below or $R^X{}_2N$ represents a cyclic alkylamine; a phosphine of the general formula $PR^X{}_2$, wherein $R^X$ is as defined below or $PR^X{}_2$ represents a cyclic phosphine; $O_jSR^X$, wherein j = 2 or 3; or $NO_r$, wherein r = 2 or 3; and derivatives thereof;
wherein $R^X$ is a substituted or unsubstituted $C_4$-$C_{24}$ aryl or a $C_3$-$C_{24}$ heteroaryl which contains one or more heteroatoms, such as B, O, N, S, Se, P, or Si; a linear or branched, substituted or unsubstituted $C_1$-$C_{20}$ alkyl; $C_2$-$C_{20}$ alkenyl or $C_2$-$C_{20}$ alkynyl; or a substituted or unsubstituted $C_3$-$C_{20}$ cycloalkyl; or derivatives thereof; or H.

**7.** The compound according to one or more of the preceding claims, **characterised in that** a is in the range of 0.01 - 5, preferably of 0.5 - 3, more preferably of 0.9 to 2.5 and most preferably about 2.

**8.** The compound according to one or more of the preceding claims, **characterised in that** b is in the range of 0.01 - 5, preferably of 0.5 - 3, more preferably of 0.9 to 2.5 and most preferably about 2.

**9.** A process for the preparation of a compound according to one or more of the preceding claims, comprising the steps of:

providing a zinc-containing salt;
adding a magnesium amide base of the formula $(R^1R^2N)$-aMg$X^1$ · bLi$X^2$,
wherein
$R^1$ and $R^2$ are independently selected from substituted or unsubstituted, linear or branched alkyl, alkenyl, alkynyl or silyl derivatives thereof, substituted or unsubstituted aryl or heteroaryl,
and wherein $R^1$ and $R^2$ can form together a ring structure, or $R^1$ and/or $R^2$ can be part of a polymer structure;
$X^1_2$ is a divalent anion or two monovalent anions that are independent from each other; $X^2$ is a monovalent anion;
a is > 0; and
b is > 0.

**10.** The process according to claim 9, **characterised in that** the magnesium amide base is dissolved in a solvent, preferably in a solvent selected from the group consisting of cyclic, linear or branched mono- or polyethers, thioethers, amines, phosphines, and derivatives thereof which contain one or more additional heteroatoms selected from O, N, S and P, preferably tetrahydrofuran (THF), 2-methyltetrahydrofuran, dibutyl ether, diethyl ether, tert-butyl methyl ether, dimethoxyethane, dioxanes, preferably 1,4-dioxane, triethylamine, ethyldiisopropylamine, dimethyl sulphide, dibutyl sulphide; cyclic and linear amides, preferably N-methyl-2-pyrrolidone (NMP), N-ethyl-2-pyrrolidone (NEP), N-butyl-2-pyrrolidone (NBP), N,N-dimethyl acetamide (DMAC); cyclic, linear or branched alkanes and/or alkenes, wherein one or more hydrogen atoms are replaced by halogen, preferably dichloromethane, 1,2-dichloroethane, $CCl_4$; urea derivatives, preferably N,N'-dimethyl propylene urea (DMPU), N,N,N',N'-tetramethyl urea; aromatic, heteroaromatic or aliphatic hydrocarbons, preferably benzene, toluene, xylene, pyridine, pentane, cyclohexane, hexane, heptane; hexamethylphosphorotriamide (HMPA), $CS_2$; or combinations thereof.

**11.** The process according to one or more of claims 9-10, **characterised in that** the addition is carried out in a temperature range of from -80 °C to 100 °C, preferably from -25 °C to 50 °C and most preferably from 0 °C to 30 °C.

**12.** The process according to one or more of claims 9-11, **characterised in that** the zinc-containing salt is dried before addition of the magnesium amide base *in vacuo* and/or at a temperature of above 50 °C, more preferably above 100 °C and even more preferably above 150 °C.

**13.** The process according to one or more of claims 9-12, **characterised in that** the concentration of the magnesium amide base upon addition to the zinc-containing salt is in the range of 0.01 - 3 M, preferably in the range of 0.1 - 1.5 M and most preferably in the range of 0.5 - 1.2 M.

**14.** The process according to one or more of claims 9-13, **characterised in that** the concentration of the resulting zinc amide base is in the range of 0.01 - 2 M, preferably in the range of 0.05 - 1 M and most preferably in the range of 0.1 - 0.6 M.

**15.** The process according to one or more of claims 9-14, **characterised in that** the zinc-containing salt is dissolved in a solvent before addition of the magnesium amide base.

## Revendications

**1.** Composé de formule générale (I)

$$(R^1R^2N)_2\text{-Zn} \cdot a\text{Mg}X^1_2 \cdot b\text{Li}X^2 \qquad \text{(I)}$$

$R^1$ et $R^2$ étant choisis indépendamment l'un de l'autre parmi l'alkyle, l'alkényle, l'alkinyle, à chaque fois substitué ou non, droit ou ramifié, ou leurs dérivés silylés, et l'aryle ou l'hétéroaryle substitué ou non, et $R^1$ et $R^2$ pouvant former conjointement une structure annulaire, ou $R^1$ et/ou $R^2$ pouvant faire partie d'une structure de polymère ; $X^1_2$ étant un anion divalent ou deux anions monovalents indépendants l'un de l'autre ; $X_2$ étant un anion monovalent ;
a > 0 ; et
b > 0.

2. Composé selon la revendication 1, **caractérisé en ce que** $R^1$ et/ou $R^2$ sont choisis indépendamment l'un de l'autre parmi l'alkyle $C_{1-20}$, de préférence l'alkyle $C_{1-10}$, encore plus de préférence l'alkyle $C_{1-6}$, et au mieux l'alkyle $C_{2-5}$, à chaque fois droit ou ramifié, substitué ou non.

3. Composé selon une ou plusieurs des revendications précédentes, **caractérisé en ce que** $R^1$ et/ou $R^2$ sont choisis indépendamment l'un de l'autre parmi des restes silylés, de préférence des restes silylés substitués par un alkyle, et notamment de préférence le triméthylsilyle, le triéthylsilyle, le triisopropylsilyle et le t-butyldiméthylsilyle.

4. Composé selon la revendication 1, **caractérisé en ce que** $R^1$ et $R^2$ forment conjointement une structure annulaire, de préférence une structure annulaire d'alcane-diyle, et notamment de préférence un 1,1,5,5-tétraméthylpentane-1,5-diyle.

5. Composé selon une ou plusieurs des revendications précédentes, **caractérisé en ce que** $X^1$ est choisi parmi le groupe constitué de : F ; Cl ; Br ; I ; CN ; SCN ; NCO ; $HalO_n$ avec n = 3 ou 4 et Hal étant choisi parmi Cl, Br, et I ; $NO_3$ ; $BF_4$ ; $PF_6$ ; ½$SO_4$ H ; un carboxylate de formule générale $R^XCO_2$ ; un thiolate de formule générale $SR^X$ ; un alcoolate de formule générale $OR^X$ , $R^XP(O)O_2$ ; $SCOR^X$ ; $SCSR^X$ ; $O_jSR^X$ avec j = 2 ou 3 ; ou $NO_r$ avec r = 2 ou 3 ; et leurs dérivés ; $R^X$ étant un aryle $C_4$-$C_{24}$ substitué ou non ou un hétéroaryle $C_3$-$C_{24}$ qui contient un ou plusieurs hétéroatomes tels que B, O, N, S, Se, P ou Si ; un alkyle $C_1$-$C_{20}$, un alkényle $C_2$-$C_{20}$ ou un alkinyle $C_2$-$C_{20}$, à chaque fois linéaire ou ramifié, substitué ou non ; ou un cycloalkyle $C_3$-$C_{20}$ substitué ou non ; ou leurs dérivés ; ou H.

6. Composé selon une ou plusieurs des revendications précédentes, **caractérisé en ce que** $X^2$ est choisi parmi le groupe constitué de : F ; Cl ; Br ; I ; CN ; SCN ; NCO ; $HalO_n$ avec n = 3 ou 4 et Hal étant choisi parmi Cl, Br, et I ; $NO_3$ ; $BF_4$ ; $PF_6$ ; ½$SO_4$ ; H ; un carboxylate de formule générale $R^XCO_2$ ; un disilazide de formule générale $(R^X{}_3Si)_2N$ ; un thiolate de formule générale $SR^X$ ; un alcoolate de formule générale $OR^X$ ; $R^XP(O)O_2$, $SCOR^X$ ; $SCSR^X$ ; une amine de formule générale $R^XNH$ ; une dialkylamine ou une diarylamine de formule générale $R^X{}_2N$ avec $R^X$ comme défini ci-dessous ou $R^X{}_2N$ représentant une alkylamine cyclique ; une phosphine de formule générale $PR^X{}_2$ avec $R^X$ comme défini ci-dessous ou $PR^X{}_2$ représentant une phosphine cyclique ; $O_jSR^X$ avec j = 2 ou 3 ; ou $NO_r$ avec r = 2 ou 3 ; et leurs dérivés ; $R^X$ étant un aryle $C_4$-$C_{24}$ substitué ou non ou un hétéroaryle $C_3$-$C_{24}$ qui contient un ou plusieurs hétéroatomes tels que B, O, N, S, Se, P ou Si ; un alkyle $C_1$-$C_{20}$, un alkényle $C_2$-$C_{20}$ ou un alkinyle $C_2$-$C_{20}$, à chaque fois linéaire ou ramifié, substitué ou non ; ou un cycloalkyle $C_3$-$C_{20}$ substitué ou non ; ou leurs dérivés ; ou H.

7. Composé selon une ou plusieurs des revendications précédentes, **caractérisé en ce que** a est compris entre 0,01 et 5, de préférence entre 0,5 et 3, encore plus de préférence entre 0,9 et 2,5 et au mieux est sensiblement égal à 2.

8. Composé selon une ou plusieurs des revendications précédentes, **caractérisé en ce que** b est compris entre 0,01 et 5, de préférence entre 0,5 et 3, encore plus de préférence entre 0,9 et 2,5 et au mieux est sensiblement égal à 2.

9. Procédé de fabrication d'un composé selon une ou plusieurs des revendications précédentes avec les étapes :

   - préparation d'un sel contenant du zinc ;
   - ajout d'une base amide au magnésium de formule $(R^1R^2N)$-$aMgX^1 \cdot bLiX^2$,

   $R^1$ et $R^2$ étant choisis indépendamment l'un de l'autre parmi l'alkyle, l'alkényle, l'alkinyle, à chaque fois substitué ou non, droit ou ramifié, ou leurs dérivés silylés, et l'aryle ou l'hétéroaryle substitué ou non, et $R^1$ et $R^2$ pouvant former conjointement une structure annulaire, ou $R^1$ et/ou $R^2$ pouvant faire partie d'une structure de polymère ; $X^1{}_2$ étant un anion divalent ou deux anions monovalents indépendants l'un de l'autre ; $X_2$ étant un anion monovalent ;
   a > 0 ; et
   b > 0.

10. Procédé selon la revendication 9, **caractérisé en ce que** la base amide au magnésium est dissoute dans un solvant, de préférence dans un solvant choisi parmi le groupe constitué de monoéthers ou polyéthers, thioéthers, amines, phosphines, à chaque fois cycliques, linéaires ou ramifiés, et leurs dérivés, qui contiennent un ou plusieurs hétéroatomes choisis parmi O, N, S et P, de préférence tétrahydrofurane (THF), 2-méthyltétrahydrofurane, dibutyléther, diéthyléther, tert-butylméthyléther, diméthoxyéthane, dioxanes, de préférence 1,4-dioxane, triéthylamine, éthyldiisopropylamine, diméthylsulfure, dibutylsulfure ; amides cycliques et linéaires, de préférence N-méthyl-2-pyrrolidone (NMP), N-éthyl-2-pyrrolidone (NEP), N-butyl-2-pyrrolidone (NBP), N,N-diméthylacétamide (DMAC) ; alcanes et/ou alcènes cycliques, linéaires ou ramifiés, un ou plusieurs atomes d'hydrogène étant remplacés par un halogène, de

préférence dichlorométhane, 1,2-dichloroéthane, $CCl_4$ ; dérivés d'urée, de préférence N,N'-diméthylpropylène urée (DMPU), N,N,N',N'-tétraméthyle urée ; hydrocarbures aromatiques, hétéroaromatiques ou aliphatiques, de préférence benzène, toluène, xylène, pyridine, pentane, cyclohexane, hexane, heptane ; hexaméthylphosphorotriamide (HMPA), $CS_2$ ; ou des combinaisons de ceux-ci.

**11.** Procédé selon une ou plusieurs des revendications 9 ou 10, **caractérisé en ce que** l'ajout s'effectue dans une plage de température allant de -80 °C à 100 °C, de préférence de -25 °C à 50 °C et au mieux de 0 °C à 30 °C.

**12.** Procédé selon une ou plusieurs des revendications 9 à 11, **caractérisé en ce que** le sel contenant du zinc est séché avant l'ajout de la base amide au magnésium, de préférence dans le vide et/ou à une température de plus de 50 °C, de préférence de plus de 100 °C et encore plus de préférence de plus de 150 °C.

**13.** Procédé selon une ou plusieurs des revendications 9 à 12, **caractérisé en ce que** la concentration de la base amide au magnésium lors de l'ajout au sel contenant du zinc est de l'ordre de 0,01 à 3 M, de préférence de l'ordre de 0,1 à 1,5 M et au mieux de l'ordre de 0,5 à 1,2 M.

**14.** Procédé selon une ou plusieurs des revendications 9 à 13, **caractérisé en ce que** la concentration de la base amide au zinc résultante est de l'ordre de 0,01 à 2 M, de préférence de l'ordre de 0,05 à 1 M et au mieux de l'ordre de 0,1 à 0,6 M.

**15.** Procédé selon une ou plusieurs des revendications 9 à 14, **caractérisé en ce que** le sel contenant du zinc est dissous dans un solvant avant l'ajout de la base amide au magnésium.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur


- **T. Bach ; S. Heuser.** *J. Org. Chem.,* 2002, vol. 67, 5789
- **A. Dondoni ; G. Fantin ; M. Foagnolo ; A. Medici ; P. Pedrini.** *J. Org. Chem.,* 1998, vol. 53, 1748
- **M. Gaudemar.** *Bull. Soc. Chim. Fr.,* 1962, 974
- **P. Knochel ; M. C. P. Yeh ; S. C. Berk.** *J. Talbert, J. Org. Chem.,* 1988, vol. 53, 2390
- **V. Snieckus.** *Chem. Rev.,* 1990, vol. 90, 879
- **M. Schlosser.** *Angew. Chem.,* 2005, vol. 117, 380
- *Angew. Chem. Int. Ed.,* 2005, vol. 44, 376
- **C.-C. Chang ; M. S. Ameerunisha.** *Coord. Chem. Rev.,* 1999, vol. 189, 199
- **J. Clayden.** Organolithiums: Selectivity for Synthesis. Elsevier, 2002
- The Preparation of Organolithium Reagents and Intermediates. **F. Leroux ; M. Schlosser ; E. Zohar ; I. Marek.** Chemistry of Organolithium Compounds. Wiley, 2004, 435
- **I. E. Kopka ; Z. A. Fataftah ; M. W. Rathke.** *J. Org. Chem.,* 1987, vol. 52, 448
- **A. Krasovskiy ; V. Krasovskaya ; P. Knochel.** *Angew. Chem. Int. Ed.,* 2006, vol. 45, 2958
- *Angew. Chem.,* 2006, vol. 118, 3024
- **Y. Kondo ; M. Shilai ; M. Uchiyama ; T. Sakamoto.** *J. Am. Chem. Soc.,* 1999, vol. 121, 3539
- **W. Clegg ; S. Dale ; R. W. Harrington ; E. Hevia ; G. W. Honeyman ; R.E. Mulvey.** *Angew. Chem. Int. Ed.,* 2006, vol. 45, 2374
- *Angew. Chem.,* 2006, vol. 118, 2434
- **W. Clegg ; S. Dale ; A. Drummond ; E. Hevia ; G. W. Honeyman ; R.E. Mulvey.** *J. Am. Chem. Soc.,* 2006, vol. 128, 7434
- **D. A. Armstrong ; W. Clegg ; S. Dale ; E. Hevia ; L. Hogg ; G. W. Honeyman ; R. E. Mulvey.** *Angew. Chem. Int. Ed.,* 2006, vol. 45, 3775
- *Angew. Chem.,* 2006, vol. 118, 3859
- **M. Hlavinka ; J. Hagadorn.** *Tett. Lett.,* 2006, vol. 47, 5049